# EUROPEAN PATENT SPECIFICATION

(11) **EP 3 700 587 B1**
(45) Date of publication and mention of the grant of the patent: **24.01.2024**
(21) Application number: 18796838.3
(22) Date of filing: 23.10.2018
(51) Int. Cl.: A61L 9/20, B01D 53/00, F24F 3/16, H01J 61/16

(54) **AN AIR TREAMENT SYSTEM AND A METHOD FOR USING SAID AIR TREAMENT SYSTEM**
LUFTBEHANDLUNGSSYSTEM UND VERFAHREN ZUR VERWENDUNG DIESES LUFTBEHANDLUNGSSYSTEMS
SYSTÈME DE TRAITEMENT D'AIR ET PROCÉDÉ D'UTILISATION DUDIT SYSTÈME DE TRAITEMENT D'AIR

(30) Priority: 24.10.2017 DK PA201770803; 16.03.2018 DK PA201870170
(43) Date of publication of application: 02.09.2020
(73) Proprietor: Airlich IP ApS, 3450 Allerød (DK)
(72) Inventor: ROSENØRN, Thomas, 3460 Birkerød (DK); INGERMAR, Jonas, 2200 København N (DK); BUTCHER, Andrew, 2700 Brønshøj (DK); CARSTENS, Cecillie Litske, 1620 København V (DK)
(74) Representative: Holme Patent A/S
(86) International application number: PCT/DK2018/050268
(87) International publication number: WO 2019/080981

(56) References cited:
- CN-U- 201 652 266
- JP-A- 2007 135 677
- RU-C1- 2 440 147
- US-A- 6 082 885
- US-A1- 2004 071 589
- US-A1- 2017 197 493

## Description

The present invention relates to an air treatment system, and a method of using said air treatment system.

It is a well known problem that air in different facilities such as homes, offices or in an industrial production rooms is contaminated with undesirable compounds and/or pollutants, e.g. volatile organic compounds, allergens and microorganisms affecting the indoor air quality and accordingly the comfort and health of the occupants in said facility.

Usually the best way to address this problem is to control or eliminate the sources of pollutants, and to ventilate the facility with clean outdoor air. The ventilation method may, however, be limited by weather conditions or undesirable levels of contaminants contained in the outdoor air. Furthermore, industrial production areas may contain pollutants which cannot be safely emitted into the surroundings.

In such situations air treatment units arranged to remove pollutants from the air are useful. Many different kind of air treatment system are known in the art, depending on the kind of pollutant in the air. However, in order to remove volatile organic compounds (VOCs) and biological material, air treatment systems utilizing UV-radiation and/or ozone have proven highly advantageous. Using this technique it is possible to sterilize the air almost completely, and attain decomposition of all organic compounds, e.g. VOCs at the same time.

Such air treatment systems are e.g. known from WO 97/34682 and WO 99/13956 in which air may be sterilized by exposing the air to UV-radiation, and organic compounds can be removed with ozone e.g. created during the UV-radiation. UV-light and ozone may be produced from the same UV-lamp, as the lamps can be arranged for emitting different wavelengths. From said patent applications, it is also known to control and regulate the ozone concentration that is emitted into the surroundings.

WO 92/10429 also relates to air cleaning by means of ozone, the ozone being produced from a UV-tube around which a medium to be sterilized is flowing, and which medium by means of deflector plates is forced to flow around a centrally placed UV-tube.

Other air treatment systems using UV-lamps are are known from RU 2440147, JP 2007 135677, US 2004/071589, US 2017/197493, US2015114912A1, CN 201652266 and US 6,082,885.

Traditionally, mercury lamps have been used for emitting UV-light in such air treatment systems. However, these lamps has the disadvantage that only a fraction of the radiation is in the UV-range, the remainder being in the visible and infrared spectrum. This means that a relatively large part of the energy used by the lamps, is not used for generating UV-light, making said lamps relatively ineffective. Furthermore, for these lamps to function, mercury has to evaporate meaning that the lamps will get very hot. Accordingly their ultra-violet outputs are significantly reduced if they are operated at e.g. room temperature. These drawbacks precludes the use of mercury lamps in some situations, or requires cooling of the air before said air e.g. can be used for air-condition and/or ventilation purposes.

Further, mercury lamps, and the mercury used in such lamps, pose a significant environmental hazard, and are accompanied by specialized handling and disposal requirements when the lamp reaches the end of its useful life.

Thus, even though there presently exists a number of air treatment systems utilising UV-light for removing pollutants from air, these solutions all require large amounts of energy, and are not effective at room temperature.

Accordingly, there remains a demand for improved systems for the removal of pollutants in air whilst offering both a reduction in energy and a substantial complete removal of pollutants in the air, e.g. VOC.

It is therefore a first aspect of the present invention to provide an air treatment system arranged for removing pollutants from the air in a fast and effective manner, using much less energy for the removal process compared to the traditional air treatment systems.

It is a second aspect of the present invention to provide an air treatment system having a compact structure, in which the pressure drop over the system is reduced, and which can be utilized, e.g. in an existing heating and/or ventilation and/or air conditioning (HVAC) system, or as a stand alone system.

It is a third aspect of the present invention to provide an air treatment system which does not require addition of expensive oxidizing agents such as hydrogen peroxide, thereby reducing both costs and space for storage facilities.

It is a fourth aspect of the present invention to provide an air treatment system arranged for removing high concentrations of pollutants at room temperatures.

It is a fifth aspect of the present invention to provide an air treatment method and system that is simple and reliable to use.

These and further aspects are achieved according to the present invention by providing an air treatment system comprising a plurality of excimer lamps, said system is arranged such that at least 90% of the contaminated air flowing through the air treatment system will be exposed to photons emitted from the excimer lamps.

Excimer lamps are quasi-monochromatic light sources available over a wide range of wavelengths in the ultraviolet (UV) and vacuum ultraviolet (VUV) spectral regions. The operation of excimer lamps is based on the formation of excited dimers (excimers). These excimer formations are unstable and will disintegrate within nanoseconds, giving up their excitation (binding) energy in the form of photons (radiation) at a characteristic wavelength.

The generated radiation (emitted photons in the UV and VUV range) will upon contact with e.g. organic contaminants in the air break down said contaminants through the process of photolysis, which is highly effective at removing different organic compounds, e.g. odours from the air. A further advantage of the emitted radiation is that it may cause oxidants, such as ozone and/or excited oxygen species, e.g. OH, O¹D, O³P to be generated from oxygen present in the air, which will proceed to oxidise organic contaminants present in the air.

The system according to the present invention is arranged such that at least 90 % of the air is subjected to the photons (UV-radiation) in the air treatment system, i.e. the photons that are released when the excimers disintegrate. This ensures that a very high degree of pollutants in the air may be removed via photolysis and/or oxidation. It is however preferred that at least 95 % of the contaminated air is subjected to photons emitted from the excimer lamps, preferably at least 99% of said contaminated air, in order to ensure a higher degree of removal of the pollutants in said air.

In a preferred embodiment according to the present invention, the excimers are produced using the rare gases, i.e. He₂, Ne₂, Ar₂, Kr₂ and Xe₂, or the rare gas halides (e.g. ArF, KrF, XeCL and XeF). However, halogenes and mercury halogen mixtures (e.g. HgCl, HGBr og HgI) are also contemplated within the scope of the present invention.

The excimers may be produced according to the present invention, by silent electrical discharge where the relevant gas for producing the excimers, e.g. xenon, is placed in a gap between two concentric quarts tubes. This technology is are well known and will not be discussed in further details in this application, however one preferred excimer lamp for use in the present invention may be a xenon lamp obtained from USHIO America Inc.

The wavelength of the emitted photons depends on the gas used to provide the excimer. This means that different wavelengths of the photons can be obtained by selecting an excimer lamp with the gas of interest. For instance, a xenon excimer lamp will generate radiation with a wavelength of 172 nm, whereas an argon excimer lamp will provide a wavelength of 129 nm and a krypton fluoride excimer lamp will provide a wavelength of 222 nm. A complete list of the relevant wavelength can be found in the literature.

The use of excimer lamps offers a number of advantages, high intensity at a defined wavelength, no-self absorption, and flexibility in the construction of the air treatment system according to the present invention.

Since only a single gas is used in each excimer lamp, the radiation output by the excimer lamps is restricted to a narrow UV wavelength range. This allows a perfect match with the absorption spectrum of the pollutants/compounds that are to be removed from the air, i.e. the excimer lamps in the air treatment system according to the invention may be selected in order to match the absorption spectrum of the pollutants in the air to be treated (contaminated air).

Furthermore, excimer lamps only generate little heat, making them highly suitable for air-condition and/or ventilation purposes, as cooling is not required before the treated air may be submitted into the surroundings.

In addition, excimer lamps have a long lifetime because the electrodes are not in direct contact with the discharge gases and will thus avoid any corrosion during the discharge process and no contamination of the excimer gas, as it is often the situation in conventional mercury lamps leading to a short operating lifetime. Finally, non-toxic materials are used in the excimer lamps and thus inherently, there is no environmental problem.

It is preferred that excimer lamps used in the present invention emit photons having a wavelength in the range between 126 nm and 240 nm, since photon emitted in this range not only will ensure a substantially complete removal of pollutants, but also that the generation of further pollutants, such as NOx, is prevented.

In one advantageous embodiment, the excimer lamps emit a wavelength of about 172 nm. The inventors of the present invention have shown that this wavelength in a very energy efficient way is capable of removing substantially all organic compounds e.g. VOC's by means of photolysis, and simultaneously sterilise the air, by inactivating microorganisms and vira. Furthermore said wavelength will also produce the oxidant ozone, that will proceed to oxidise organic contaminants present in the air.

However, other wavelengths are is also preferred within the scope of the present invention. As an example it can be mentioned that wavelengths around 185 nm will generate ozone, and wavelengths around 222 nm have proven to be effective in destroying double bonds e.g. C=C and C=O. KrI excimer lamps will provide photons with a wavelength of 185 nm and KrCl will emit photos having a radiation peak at 222 nm. A radiation peak around 222 nm will, if humidity is present in air to be treated, also provide a photo-induced production of hydrogen peroxide (H₂O₂). Since hydrogen peroxide is a strong oxidation agent (as is ozone) this will further ensure an effective removal of organic pollutants.

In a preferred embodiment (not part of the invention), the system comprises an air treatment housing in which the excimer lamps are accommodated/placed. Said air treatment housing is preferably arranged such that contaminated air flowing though said housing will flow over the surface of the excimer lamps and/or close to the surface of said lamps, thereby ensuring that at least 90% of the contaminated air flowing through the air treatment system will be exposed to photons emitted from the excimer lamps.

It is accordingly preferred that the excimer lamps are placed in close proximity to each other. The inventors of the present invention have shown that this is achieved when the direct (i.e. shortest) distance (taken in a cross-section) between the surface of two adjacent excimer lamps is below 4 cm, and preferably even lower, such as below 2 cm or more preferred below 1 cm. The cross-section is preferably the same over substantially the entire air treatment housing.

It is also preferred that the excimer lamps are placed close to the walls of the air treatment housing, such that air passing close to said walls will also be exposed to the emitted photos. The direct distance between the walls of the housing and the surface of the excimer lamps is preferably below 2 cm, preferably below 1.5 cm and more preferred below 1 cm.

Thus, in one embodiment (not part of the invention) the air treatment housing will comprise a plurality of excimer lamps placed in close proximity to each other and the walls of said housing. Such an arrangement will effectively ensure that the emitted photons will get in contact with substantially all contaminants in the air and effectively clean/treat said air, as the emitting photons will initiate an photolysis process in the air, and/or ensure that ozone will be generated from oxygen present in the air.

The excimer lamps are preferably distributed evenly in said air treatment housing, e.g. in a number of substantially parallel rows and columns, and/or arrays, providing a matrix with a plurality of substantially uniformly distributed and parallel excimer lamps.

The air treatment system comprises a plurality of identical air treatment units, wherein each unit comprises a single excimer lamp.

Utilising a number of smaller air treatment units instead of an air treatment housing in which the plurality of excimer lamps is placed, provides a high degree of flexibility to design in different geometries. For instance, the air treatment units may be combined into different shapes thereby ensuring that the system according to the present invention can be adapted to fit into different existing air treatment systems, e.g. conventional ventilation systems.

It is preferred that the air treatment units are arranged for being assembled into a combined unit, e.g. by comprising means for joining the units together or by packing the units into an outer casing having the desired shape and dimension, e.g. to match an existing ventilation system.

The air treatment units are arranged such that the direct distance between the surface of an excimer lamp and the inner wall of the air treatment unit (taken in a cross-section) is 2 cm or lower, e.g. 1.5 cm or even more preferred 1.0 cm. This will effectively ensure that substantially all, and at least 90% of the air passing though an air treatment unit will be exposed to photons emitted from the excimer lamps, when the unstable exited excimer disintegrate, i.e. the contaminated air will flow over the surface of the excimer lamps and/or so close to the surface of said lamps that at least 90% of the contaminated air will be exposed to photons emitted from the excimer lamps

The cross-sectional shape of the air treatment units according to the present invention is in the form of a hexagon. This means that the air treatment units can be fitted closely together without any wasted space between the units, i.e. the units can fill a flat plane completely. The direct distance between the surface of the excimer lamp and the inner wall of the air treatment unit (taken in a cross-section) may in these situation be measured to the corners of the hexagon.

The respective air treatment units are constructed in such a way that adjacent air treatment units share at least a part of the wall structures of one or more adjacent air treatment units, as this effectively will reduce the materials required to provide the final construction/assembly of air treatment units. Since an air treatment unit having a hexagonal cross sectional shape will require the least total length of wall, compared with triangles or squares of the same area, air treatment units having a cross sectional shape in the form of a hexagon are preferred in this respect, and will accordingly provide a honeycomb-like structure.

One main advantage of using air treatment units is that fewer excimer lamps are required in order to obtain a high efficiency of the system according to the invention. As an example it can be mentioned that if a plurality of air treatment units are placed in a honeycomb structure, the distance between the surface of two excimer lamps in two adjacent air treatment units will be longer than the distance between the surfaces of two adjacent excimer lamps accommodated in e.g. an air treatment housing. Accordingly, fewer excimer lamps are required in an air treatment system comprising a plurality of air treatment units, compared to an air treatment system comprising an air treatment housing with substantially similar outer dimensions as the assembly of air treatment units. However, since at least 90% of the air passing though an air treatment unit will be exposed to photons emitted from the excimer lamps in both constructions, the efficiency of the system will be the same.

Even though an air treatment unit comprising a plurality of air treatment units provides a more complex construction than a similar system with an air treatment housing, the lower cost associated with the reduced number of required excimer lamps, provides a significant reduction in the cost for the air treatment system using air treatment units.

In a preferred embodiment the excimer lamps are elongated cylindrically excimer tubes having a longitudinal axis arranged in the flow direction of the air treatment unit, i.e. the air to be treated will flow along the length of the excimer tubes. This will also ensure that the contaminated air is exposed to photons in the complete length of the air treatment unit thereby fully utilising the capacity of the air treatment system.

Cylindrical excimer lamps are well known in the art, and typically consist of two co-axial tubes that are made of a dielectric material, usually quartz, and contain a rare gas or a mixture of rare gases/halogens at close to atmospheric pressure. The imposition of an electrical potential across the two dielectric barriers leads to the formation of the excited molecular complex, i.e. the excimer.

It is preferred that the contaminated air stream is exposed to the emitted photons for a period of at least 1 ms, as this has proven to be sufficient for removing pollutants, e.g. VOCs, microorganisms, and odours, in domestic buildings. The air treatment unit may accordingly have a size and dimension in which this can be provided, taking into consideration the flow rate of the air.

In a preferred embodiment the air treatment system comprises a plurality of excimer lamps arranged for emitting the same wavelength, thereby providing a simple and relative inexpensive system. It is in this respect preferred that the emitted wavelength is about 172 nm, as the inventors have shown that this wavelength is capable of removing substantially all pollutants in air from domestic building, as well as from similar environments.

However, if the contaminated air comprises several different kinds of pollutants, the air treatment system according to the invention may comprises at least a first group of excimer lamps arranged for emitting a first wavelength, and a second group of excimer lamps arranged for emitting a second wavelength, and wherein the first and the second wavelength are J Z different. This will ensure that air passing though the system according to the invention will be exposed to photons having different wavelengths. For instance, if the first group of excimer lamps emits a wavelength of 172 nm (initiating photolysis) and the second group emits a wavelength of 222 nm (generates H₂O₂), contaminated air will be subjected to both wavelengths providing a more effective treatment.

Thus, using at least two groups of excimer lamps having different wavelengths each restricted to a narrow UV wavelength range, ensures that the air treatment system can be constructed to meet different demands, depending on the pollutants/compounds in the contaminated air. For instance, air to be treated from an industrial kitchen may require a different wavelength or combination of wavelengths than an air treatment system aiming at treating indoor air from a family home.

The system according to the invention may comprise additional groups of excimer lamps wherein each group emits a wavelength which is different from the wavelength emitted from the other groups. The number of groups can be varied, but may be three, four, five etc. depending on the pollutants in the air to be treated.

The excimer lamps from the respective groups may in one embodiment be uniformly mixed with excimer lamps from one or more of the remainder groups, in order to ensure that air passing though the air treatment housing is exposed to the photons with the different wavelengths and/or the oxidants generated from said photons.

Alternatively the air treatment system comprises at least a first air treatment zone comprising excimer lamps arranged for emitting a first wavelength, and a second air treatment zone comprising excimer lamps arranged for emitting a second wavelength, and wherein said first and the second wavelength is different, thereby ensuring that the contaminated air will pass through different air treatment zones in the air treatment system, and wherein the contaminated air in each zone is subjected to an individual and restricted wavelength.

This embodiment has the advantage that if the air to be treated contains certain pollutants/compounds that may negatively influence the treatment in subsequent zones, said pollutants may be removed in the first zone, thereby optimising the treatment process. Alternatively, the one or more zones may be arranged to emit more than one wavelength, and may e.g. comprise two or more groups of excimer lamps emitting different wavelengths, as discussed earlier.

Further treatment zones, e.g. three, four etc. are also contemplated within the scope of the present invention.

In order to ensure a fast and effective treatment of the contaminated air, it is preferred that the air treatment unit does not comprise any means e.g. baffles and the like, that will change the flow direction of the air in said unit. This will effectively reduce the pressure drop over the system compared to systems using such means.

In order to provide an energy efficient air treatment the system according to the invention may be arranged for operating the excimer lamps with a pulse repetition frequency, i.e. the excimer lamps are switched on and off. This will not only prolong the lifetime of the excimer lamps, but it has also been shown that this will significantly increase the efficiency of conversion of power to excimer formation and, consequently, increase the efficiency of conversion of applied power to photons.

Preferably, the pulses are of a short duration, desirably about 100 ms or less (such as about 100 ns), with a carrier frequency between 10 HZ and 100 kHz, such as about 20kHZ. Preferably, the pulsed potential has a duty cycle such that the potential is on about 75 percent or less of the total time, more desirably about 50 percent or less of the total time, and most desirably about 25% or less of the total time.

One very important consequence of the brief duration of the pulse is that it minimizes the power used for generating the photons. Furthermore, the use of relatively short pulse duration avoids arcing and provides for operation at a higher potential and thus higher efficiency of the excimer lamps.

The pulsed operation of the excimer lamps may be achieved using any means, e.g. by simply pulsating the power supply to the excimer lamps or by using a potential source arranged for applying the pulsed potential or by Pulse Width Modulation (PWM) .

The number of excimer lamps may vary depending on the intended use. However, it is preferred that the air treatment system according to the invention comprises at least 50 excimer lamps, preferably at least 100 excimer lamps, and even more preferred at least 500 excimer lamps. However, larger numbers such as a 1000 excimer lamps or smaller numbers, such as 2, 4 or 10 are also contemplated within the scope of the present invention.

In order to ensure an effective use of the photons in the air treatment unit, it is preferred that at least the inner walls of said unit are made of a reflective material arranged for reflecting at least a portion of photons emitted by the excimer lamp. Preferably the reflective material is at least 80 percent reflective - this may be achieved using materials such as stainless steel.

The system according to the invention may comprise means, e.g. a least one ventilator, arranged for sending the air to be treated past/over the plurality of excimer lamps in the air treatment system, and may be powered by any conventional means.

In a preferred embodiment according to the present invention the air treatment system according to the invention is incorporated into a heating, ventilation or air condition system (HVAC). In is in this respect advantageous is the system according to the invention is arranged for being retrofitted into an existing HVAC system at the relevant facilities, thereby reducing cost for expensive installation of new equipment. In these situations the means for putting the air in motion may already be part of the HVAC systems.

Even though the residuals from the processes taken place in the air treatment system consist mainly of carbon dioxide and water, it may in some situations be advantageous to combine the photooxidation process with additional treatment methods if required, e.g. electrostatic precipitation, mechanical filtration, catalysis or non-thermal plasma processes, before emitting the treated air into the environment. This is especially relevant if the air to be treated contains certain pollutants which will not (or can not) be degraded by the photolysis/photooxidation initiated by the photons emitted by the excimer lamps. These processes are also known in the art, and will not be described in further details in this application.

In addition to treating contaminated air, the air treatment system according to the present invention can be modified in order to treat/clean one or more filter devices. Such filter devices may in a preferred embodiment be an air filter such as a high efficiency particulate air (HEPA) filter, a carbon filter, and/or an electrostatic precipitator (ESP).

The respective filter devices will, when air passes though the filter devices, collect particles e.g. organic particles and biological material in the form of microorganisms, viruses and mould. Said particles will eventually clog the filter and reduce the devices efficiency. As the particle load increases, so will the resistance to flow and hence the pressure drop across the filter device. Since most filter devices cannot be cleaned to remove the particles to a desired level, the respective filter devices must be replaced frequently, often at significant costs.

However the inventors of the present invention have has found that the air treatment system according to the invention may be modified in order to inactivate/kill microorganisms and viruses trapped in said filter device and/or as to treat/decompose trapped organic particles, and accordingly clean said filter devices, at least to a substantial degree thereby prolonging the time before the filter device has to be replaced.

The modified air treatment system is preferably arranged such that the entire surface of the filter device is subjected to photons emitted from the excimer lamps, i.e. the number, orientation and position of the excimer lamps in the modified air treatment system are selected in order to ensure that at least 90 %, preferably at least 95%, and even more preferred at least 99% of the surface of the filter device(s) is exposed to photons emitted from the excimer lamps.

In order to obtain this effect the filter device(s) to be treated is preferably placed in close proximity to the one or more excimer lamps, i.e. the shortest distance (taken in cross-section) between the surface of the excimer lamp(s) closest to the filter device, and the surface of the filter device, is about 2 cm, preferably lower, e.g. 1.5 cm or even more preferred 1.0 cm. In one embodiment excimer lamps are placed on both sides of the filter device, thereby ensuring that substantially the entire filter device is subjected to the emitted photons, thereby effectively cleaning/treating the filter device.

The generated radiation emitted from the excimer lamps will upon contact with the particles, e.g. microorganisms or organic particles trapped in the filter device, kill/inactivate J 2 the microorganisms, vira etc, and/or degrade the organic substances through the process of photolysis. The specific action will depend on the emitted radiation by the excimer lamps. For instance if the radiation is about 254 nm, microorganisms will be killed or inactivated since the radiation will destroy nucleic acids thereby disrupting the microorganisms DNA, whereas wavelengths in the area of about 220 nm are capable of destroying double bonds in organic pollutants. Some wavelengths e.g. wavelengths in the area of about 172 nm are capable of both decomposing organic compounds and inactivating microorganisms and vira and such wavelengths are accordingly preferred in the present invention. Alternatively, a number of excimer lamps with different wavelengths may be used if different pollutants/compounds are to be removed from the filter devices(s), and/or the excimer lamps may be combined with conventional UV producing lamps, e.g. mercury- or LED lamps if relevant.

In a preferred embodiment the filter device to be treated/cleaned is incorporated into an air treatment system according to the invention. This will ensure that the air passing though the system will be subjected to both a photooxidation process as well as a filtration step. As the filter device also is placed in proximity to the excimer lamps, said filter device will automatically be cleaned/treated by the radiation from the excimer lamps thereby providing a simple and effective air treatment system.

The present invention also relates to a method of treating contaminated air and/or a filter device using the air treatment unit according to the present invention, and wherein at least 90%, preferably at least 95 % or more preferred 99%, of the contaminated air stream flowing through the air treatment system is exposed to photons emitted from the excimer lamps. This will, as already described provide an effective degradation, and accordingly removal, of the pollutants in the contaminated air.

Since the excimer lamps only generate very little heat, said method may be effected at room temperature, i.e. around 10 - 50 °C, preferably around 20 - 25 °C.

The invention will be explained in greater detail below, describing only exemplary embodiments of the exhaust gas treatment system and method with reference to the drawings, in which
Fig. 1 schematically shows a section of a first embodiment of an air treatment system (not part of the invention),
Fig 1a, shows a cross-sectional view of the embodiment of fig. 1,
Fig. 2 schematically shows a section of a second embodiment of an air treatment system (not part of the invention),
Fig 2a, shows a cross-sectional view of the embodiment of fig. 2,
Fig. 3 shows a section of cross-sectional view of an embodiment of the air treatment system,
Fig. 4 schematically shows a section of a fourth embodiment an air treatment system (not part of the invention),
Fig. 5 schematically shows a fifth embodiment of an air treatment system (not part of the invention), and
Fig 6 shows a cross-sectional view of a modified embodiment of an air treatment system according to the invention.

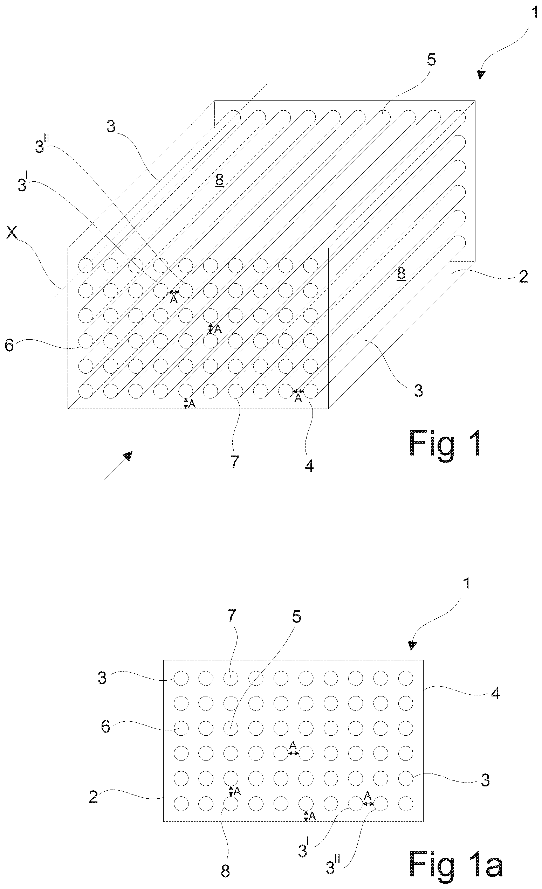

Fig. 1 shows a first simplified embodiment of an air treatment system 1 according to the invention. Said system 1 comprises an air treatment housing 2 in which a plurality of excimer lamps 3 are placed in close proximity to each other and to the walls 4 of the housing.

The excimer lamps 3 are elongated cylindrically excimer tubes 5 having a longitudinal axis X arranged in the flow direction of the air treatment housing (illustrated by an arrow), i.e. the air will flow along the length of the excimer tubes 5, thereby ensuring that the air flowing into the system 1 has the longest possible contact time with the excimer lamps 3 and accordingly the emitted photons.

The excimer lamps 3 are evenly distributed in the air treatment housing 2 in a number of parallel rows 6, and columns 7, providing a matrix with a plurality of uniformly distributed and parallel excimer lamps 3 as e.g. illustrated fig. 1a.

The direct (i.e. shortest) distance A (seen in cross-section) between the surface 8 of two adjacent excimer lamps 3',3", as well as to the walls 4 of the housing is identical throughout the housing 3. In the embodiment shown said distance A is about 2 cm, however similar designs are contemplated within the scope of protection in which the distance A is higher, e.g. below 4 cm, or lower, e.g. 1.5 cm or even more preferred about 1 cm.

Having a construction of the system 1 according to the invention, in which the contaminated air is forced to flow over and/or very close to the surface 8 of the excimer lamps 3, will ensure that at least 90% of the contaminated air flowing through the air treatment system 1 will be exposed to photons emitted from the excimer lamps 3.

Said photons are emitted when the generated excimers disintegrate within nanoseconds, and the photons will initiate the photolysis process in which chemical compounds present in the air are broken down by the photons. Since indoor air in domestic facilities mainly consists of organic compounds, the air treatment system 1 will ensure that said compounds in a simple and energy efficient way are decomposed into carbon dioxide and water which safely can be emitted into the surroundings.

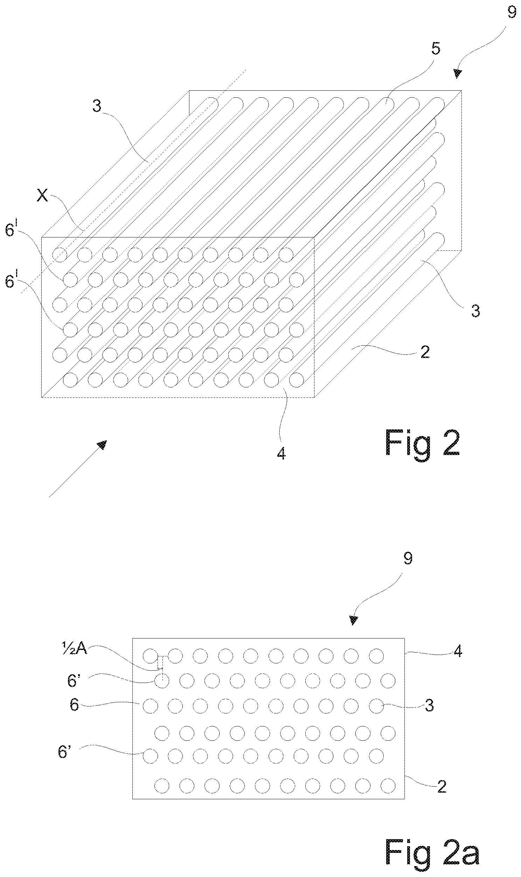

Fig. 2 shows a second embodiment of the air treatment system 9 of the present invention. The second embodiment 9 corresponds basically to the first embodiment 1, and the function is identical to the first embodiment, but where the excimer lamps 3 were distributed evenly in a number of parallel rows 6 and columns 7 in the first embodiment, every second row 6' has been displaced half the distance A in the second embodiment, providing an alternating matrix, as best illustrated in fig. 2a.

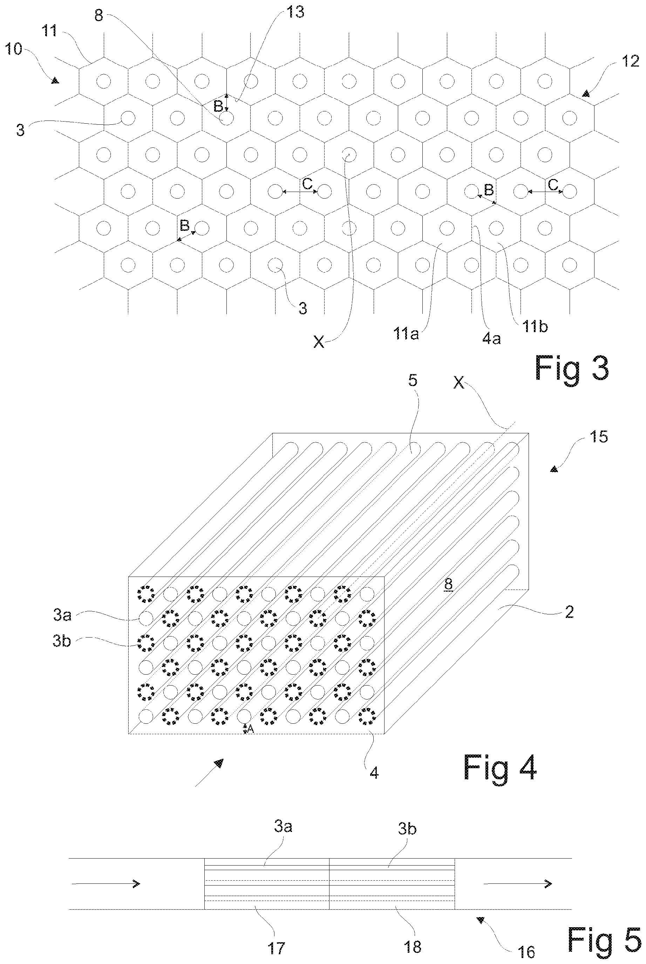

A third embodiment 10 of the air treatment system is shown in fig.3 in which a plurality of hexagon shaped air treatment units 11 is combined into a single construction 12. Each air treatment unit comprises a single excimer lamp 3 in the form of an elongated excimer tube. Said air treatment units are constructed in such a way that adjacent air treatment units, e.g. 11a, 11b share at least a part of the wall structures 4a, of one or more adjacent air treatment units, thereby providing a honeycomb structure 13.

Each excimer tube has a longitudinal axis X arranged in the flow direction of the air treatment unit, i.e. the air will flow along the length of the excimer tubes.

Utilising a number of smaller air treatment units 11 instead of an air treatment housing 2, provides a high degree of flexibility to design in different geometries, i.e. when the air treatment units 12 have been assembled/constructed into a final construction, e.g. the honeycomb structure 12 shown in fig. 3, said final construction can be custom made to fit into different existing air treatment systems, e.g. conventional HVAC systems.

The distance B between the surface 8 of an excimer lamp 3 and one of the corners 14 of treatment unit 11 is about 2 cm, preferably lower, e.g. 1.5 cm or even more preferred 1.0 cm. This will effectively ensure that substantially all, and at least 90% of the air passing though an air treatment unit will be exposed to photons emitted from the excimer lamps 3.

When a honeycomb system of a plurality of air treatment units, as shown in fig. 3, the distance B being 2 cm, is compared with an air treatment housing, as e.g. shown in fig. 1, and in which the distance A also is 2 cm, it is clear that distance C between the surface of two excimer lamps 3',3' ' in two adjacent air treatment units 11a, 11b is about 4 cm, i.e. twice the distance of two adjacent excimer lamps 3',3" in the air treatment housing 2.

Thus, since fewer excimer lamps are required in an air treatment system 10 comprising a number of air treatment units 11 compared to a air treatment system 1 comprising an air treatment housing 2 having similar outer dimensions, the use of air treatment units will effectively reduce costs without making compromises as to the efficiency of the air treatment system.

Excimer lamps 3 are restricted to a narrow UV wavelength range, which allows for a perfect match with the absorption spectrum of the compounds that are to be removed from the air. However, some wavelengths will also produce other compounds, e.g. ozone or hydrogen peroxide, which may aid in the treatment process since they are strong oxidation agents.

In order to ensure that this is effectively utilised in the present invention, a fourth embodiment 15 shown in fig. 4. Said embodiment corresponds in principal to the embodiment shown in fig. 1, but instead of comprising a plurality of identical excimer lamps 3, the fourth embodiment 15 comprises a first group of excimer lamps 3a, arranged for emitting a first wavelength, and a second group of excimer lamps 3b arranged for emitting a second wavelength different from the first wavelength. The excimer lamps 3a from the first group are uniformly mixed (evenly distributed) with excimer lamps 3b from the first group in the housing, in order to ensure that air passing though the air treatment housing 2 is exposed to photons with both wavelengths.

Since the first and the second wavelengths are different, the air passing though the housing will be exposed to photons having two wavelengths. Thus, using two groups of excimer lamps 3a,3b, having different wavelength, each restricted to a narrow UV wavelength range, ensures that the desired result easily can be adjusted depending on the expected compounds in the contaminated air. For instance, if the first group of excimer lamps 3a emits a wavelength of 172 nm, said group is specifically directed to initiating the photolysis process, and if the second group of excimer lamps 3b emits a wavelength of 185 nm, said second group will be specifically arranged for generating ozone. The combination of both wavelengths provides a highly effective treatment system 15 according to the invention.

It will be understood, that photons emitted with a wavelength of both 172 nm and 185 nm will be capable of initiating photolysis and the generation of ozone, however said photons will not provide optimal photon intensities (yields) for the specific purpose, and it is therefore preferred to combine different groups of excimer lamps.

A person skilled in the art will understand that further groups of excimer lamps may be incorporated and preferably evenly distributed with the other groups, and that the embodiment shown in fig. 2. may comprise two or further groups of excimer lamps emitting different wavelengths.

As an alternative to using two or more groups of excimer lamps, a fourth embodiment 16 of the air treatment system shown in fig. 5, may comprise a first air treatment zone 17 comprising excimer lamps 3a arranged for emitting a first wavelength, and a second air treatment zone 18 comprising excimer lamps 3b arranged for emitting a second wavelength, and wherein first and the second wavelengths are different. This construction will ensure that the contaminated air will pass through different air treatment zones in the air treatment system, and wherein the contaminated air in each zone 17,18 is subjected to an individual and restricted wavelength. This embodiment has the advantage that if the air to be treated contains certain pollutants/compounds that may negatively influence the treatment in subsequent zones, said pollutants may be removed in the first zone, thereby optimising the treatment process.

The air treatment system according to the invention may be arranged for operating the excimer lamps with a pulse repetition frequency (not shown), thereby prolonging the lifetime of the excimer lamps, and increase the efficiency of conversion of power to excimer formation and, consequently, increase the efficiency of conversion of applied power to photons.

The pulsed operation of the excimer lamps may be achieved using any means (not shown), e.g. by simply pulsating the power supply to the excimer lamps or by using a potential source arranged for applying the pulsed potential.

In a preferred embodiment according to the present invention the air treatment system according to the invention is incorporated into a heating, ventilation or air condition system (HVAC).

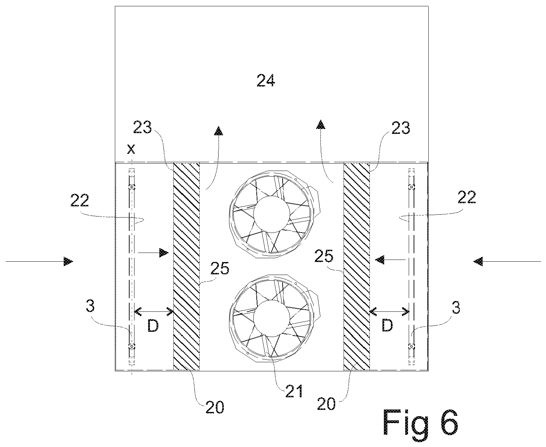

Fig. 6 shows a cross section of a modified embodiment of the air treatment system 19 according to the invention. In said embodiment two filter devices 20 are placed downstream of the excimer lamps 3, seen in the flow direction (illustrated by arrows), and two fans 21 are arranged to draw air though the system.

The direct (i.e. shortest) distance D (seen in cross-section) between the surface 22 of the excimer lamps 3 and the surface 23 of the filter device 20 is substantially identical throughout the system. In the embodiment shown said distance A is about 2 cm, however similar designs are contemplated within the scope of protection in which the distance D is higher, e.g. below 4 cm, or in which the distance is lower, e.g. 1.5 cm or even more preferred about 1 cm.

After the air has passed though the filter devices said air is in the embodiment shown exposed to a further treatment by a treatment device 24. Said treatment may be any desired treatment e.g. a further filter device or a catalytic treatment.

The number of excimer lamps 3 is selected in order to ensure that substantially the entire surface 23 of the filter devices 20 (facing the excimer lamps) is subjected to the emitted photons and the number of excimer lamps 3 is accordingly depending on the surface area of the filter devices. In the embodiment shown the longitudinal axis (X) of the excimer lamps 3, is arranged perpendicular to the flow direction, as this will ensure that the longitudinal surface of the excimer lamps 3 has the shortest distance D to the surface of a flat filter device. However, the orientation, placement and number of excimer lamps 3 depends on the construction and the placement of the filter device.

In a further embodiment excimer lamps 3 may also be placed on the opposite side 25 of the filter device 20, i.e. between the filter devices and the fans 21, as this will ensure that the filter device is exposed to the photos emitted by said excimer lamps on both sides.

Because of the unique construction of the air treatment system according to the invention in which a plurality of excimer lamps provides large emission area in which high energy ultraviolet (UV) photons is provided at low cost. The system can be designed with different wavelengths, making the system a simple and inexpensive air treatment system both for use in large-area industrial applications and for domestic uses.

## Claims

1. An air treatment system (1;9;10;15;16) comprising a plurality of excimer lamps (3), and a plurality of air treatment units (11), wherein each air treatment unit comprises a single excimer lamp (3) and **characterized in that** the air treatment units (11) have a tubular prism shape having a cross-sectional shape of a hexagon and wherein a plurality of air treatment units are assembled into a honeycomb-like structure, and wherein each excimer lamp has the form of an elongated tube and is positioned equidistant from the corners of said hexagon, whereby the distance (B) between the surface of the excimer lamp and the inner wall of the air treatment unit (4) is 2 cm or lower, wherein said distance is measured in the cross-section of said air treatment units from the surface of the lamp to the corners of the hexagon

2. An air treatment system (1;9;10;15;16) according to claim 1, wherein the excimer lamps (3) are arranged for emitting a wavelength in the range between 126 nm and 240 nm, preferably about 172 nm.

3. An air treatment system (10) according to claim 1 or 2, wherein the distance (B) between the surface of the excimer lamp and the inner wall of the air treatment unit (4) is 1.5 cm or 1.0 cm, wherein said distance is measured in the cross-section of said air treatment units from the surface of the lamp to the corners of the hexagon.

4. An air treatment system (1;9;10;15;16) according to any of the preceding claims, wherein the excimer lamps (3) are elongated cylindrically excimer tubes (5) having a longitudinal axis (X) arranged in the flow direction of the air treatment housing (2) or the air treatment unit (11) .

5. An air treatment system (15) according to any of the claims, wherein the air treatment system (15) comprises at least a first group of excimer lamps (3a) arranged for emitting a first wavelength, and a second group of excimer lamps (3b) arranged for emitting a second wavelength, and wherein the first and the second wavelengths are different.

6. An air treatment system (16) according to any of the preceding claims, the air treatment system (16) comprising at least a first air treatment zone (17) comprising excimer lamps (3a) arranged for emitting a first wavelength, and a second air treatment zone (18) comprising excimer lamps (3b) arranged for emitting a second wavelength, and wherein first and the second wavelengths are different.

7. An air treatment system (1;9;10;15;16) according to any of the claims 1 - 4, wherein all the excimer lamps (3) in the air treatment system (1;9;10;15;16) are arranged for emitting the same wavelength.

8. An air treatment system (1;9;10;15;16) according to any of the preceding claims, wherein the air treatment system (1;9;10;15;16) is arranged for operating the excimer lamps (3) with a pulse repetition.

9. An air treatment system (1;9;10;15;16) according to claim 8, wherein the pulses are of a short duration, about 100 ms or less, with a carrier frequency between 10 Hz and 100 kHz, such as about 20kHz.

10. An air treatment system (19) according to any of the preceding claims, modified in that the air treatment system comprises at least one filter device (20), said system (19) is arranged such that substantially the entire surface of the filter device(s) facing the excimer lamps is subjected to photons emitted from the excimer lamps.

11. An air treatment system (19) according to claim 10 wherein the direct distance (D) between the surface of the excimer lamps (22) and the surface of the at least one filter device (23) facing the excimer lamps (3) is 2 cm or lower, e.g. 1.5 cm or even more preferred 1.0 cm.

12. A method of treating an contaminated air stream using the air treatment system (1;9;10;15;16) according to any of the claims 1 - 11,

13. A method according to claim 12, wherein the contaminated air stream is exposed to photons for a period of at least 1 ms.

14. A method according to claim 12 or 13, wherein the treatment is effected at room temperature, i.e. around 10 - 50 °C, preferably around 20 - 25 °C.

15. A heating and/or ventilation and/or air conditioning system comprising the air treatment system (1;9;10;15;16) according to any of the claims 1 - 11.

16. Use of an air treatment system (1;9;10;15;16) according to any of the claims 1 - 11 for treating a contaminated air steam comprising pollutants in the form of organic compounds, such as VOCs.

## Patentansprüche

1. Luftbehandlungssystem (1; 9; 10; 15; 16), umfassend eine Mehrzahl von Excimer-Lampen (3) und eine Mehrzahl von Luftbehandlungseinheiten (11), wobei jede Luftbehandlungseinheit eine einzelne Excimer-Lampe (3) umfasst und **dadurch gekennzeichnet ist, dass** die Luftbehandlungseinheiten (11) eine rohrförmige Prismenform mit einer Querschnittsform eines Sechsecks aufweisen und wobei eine Mehrzahl von Luftbehandlungseinheiten zu einer wabenartigen Struktur zusammengesetzt sind, und wobei jede Excimer-Lampe die Form eines länglichen Rohrs aufweist und äquidistant von den Ecken des Sechsecks positioniert ist, wobei der Abstand (B) zwischen der Oberfläche der Excimer-Lampe und der Innenwand der Luftbehandlungseinheit (4) 2 cm oder weniger beträgt, wobei der Abstand im Querschnitt der Luftbehandlungseinheiten von der Oberfläche der Lampe zu den Ecken des Sechsecks gemessen wird.

2. Luftbehandlungssystem (1; 9; 10; 15; 16) nach Anspruch 1, wobei die Excimer-Lampen (3) zum Emittieren einer Wellenlänge im Bereich zwischen 126 nm und 240 nm, vorzugsweise etwa 172 nm, angeordnet sind.

3. Luftbehandlungssystem (10) nach Anspruch 1 oder 2, wobei der Abstand (B) zwischen der Oberfläche der Excimer-Lampe und der Innenwand der Luftbehandlungseinheit (4) 1,5 cm oder 1,0 cm beträgt, wobei der Abstand im Querschnitt der Luftbehandlungseinheiten von der Oberfläche der Lampe zu den Ecken des Sechsecks gemessen wird.

4. Luftbehandlungssystem (1; 9; 10; 15; 16) nach einem der vorhergehenden Ansprüche, wobei die Excimer-Lampen (3) längliche zylindrische Excimer-Röhren (5) mit einer Längsachse (X) sind, die in der Strömungsrichtung des Luftbehandlungsgehäuses (2) oder der Luftbehandlungseinheit (11) angeordnet sind.

5. Luftbehandlungssystem (15) nach einem der **vorhergehenden** Ansprüche, wobei das Luftbehandlungssystem (15) mindestens eine erste Gruppe von Excimer-Lampen (3a), die zum Emittieren einer ersten Wellenlänge angeordnet sind, und eine zweite Gruppe von Excimer-Lampen (3b), die zum Emittieren einer zweiten Wellenlänge angeordnet sind, umfasst und wobei die erste und die zweite Wellenlänge unterschiedlich sind.

6. Luftbehandlungssystem (16) nach einem der vorhergehenden Ansprüche, wobei das Luftbehandlungssystem (16) mindestens eine erste Luftbehandlungszone (17), die Excimer-Lampen (3a) umfasst, die zum Emittieren einer ersten Wellenlänge angeordnet sind, und eine zweite Luftbehandlungszone (18), die Excimer-Lampen (3b) umfasst, die zum Emittieren einer zweiten Wellenlänge angeordnet sind, umfasst und wobei die erste und die zweite Wellenlänge unterschiedlich sind.

7. Luftbehandlungssystem (1; 9; 10; 15; 16) nach einem der Ansprüche 1-4, wobei alle Excimer-Lampen (3) in dem Luftbehandlungssystem (1; 9; 10; 15; 16) zum Emittieren der gleichen Wellenlänge angeordnet sind.

8. Luftbehandlungssystem (1; 9; 10; 15; 16) nach einem der vorhergehenden Ansprüche, wobei das Luftbehandlungssystem (1; 9; 10; 15; 16) zum Betreiben der Excimer-Lampen (3) mit einer Pulswiederholung angeordnet ist.

9. Luftbehandlungssystem (1; 9; 10; 15; 16) nach Anspruch 8, wobei die Pulse von kurzer Dauer sind, vorzugsweise etwa 100 ms oder weniger, mit einer Trägerfrequenz zwischen 10 Hz und 100 kHz, wie etwa 20 kHz.

10. Luftbehandlungssystem (19) nach einem der vorhergehenden Ansprüche, dadurch modifiziert, dass das Luftbehandlungssystem mindestens eine Filtervorrichtung (20) umfasst, wobei das System (19) so angeordnet ist, dass im Wesentlichen die gesamte Oberfläche der Filtervorrichtung(en), die den Excimer-Lampen zugewandt ist, von den Excimer-Lampen emittierten Photonen ausgesetzt ist.

11. Luftbehandlungssystem (19) nach Anspruch 10, wobei der direkte Abstand (D) zwischen der Oberfläche der Excimer-Lampen (22) und der Oberfläche der mindestens einen Filtervorrichtung (23), die den Excimer-Lampen (3) zugewandt ist, 2 cm oder weniger beträgt, z. B. 1,5 cm oder noch bevorzugter 1,0 cm.

12. Verfahren zum Behandeln eines verunreinigten Luftstroms unter Verwendung des Luftbehandlungssystems (1; 9; 10; 15; 16) nach einem der Ansprüche 1-11.

13. Verfahren nach Anspruch 12, wobei der verunreinigte Luftstrom für einen Zeitraum von mindestens 1 ms Photonen ausgesetzt ist.

14. Verfahren nach Anspruch 12 oder 13, wobei die Behandlung bei Raumtemperatur erfolgt, d. h. um 10-50 °C, vorzugsweise um 20-25 °C.

15. Heizungs- und/oder Lüftungs- und/oder Klimatisierungssystem, umfassend das Luftbehandlungssystem (1; 9; 10; 15; 16) nach einem der Ansprüche 1-11.

16. Verwendung eines Luftbehandlungssystems (1; 9; 10; 15; 16) nach einem der Ansprüche 1-11 zum Behandeln eines verunreinigten Luftstroms, umfassend Schadstoffe in Form von organischen Verbindungen, wie etwa VOCs.

## Revendications

1. Un système de traitement de l'air (1; 9; 10; 15; 16) comprenant une pluralité de lampes à excimères (3) et une pluralité d'unités de traitement de l'air (11), dans lequel chaque unité de traitement de l'air comprend une seule lampe à excimère (3) et **caractérisé en ce que** les unités de traitement de l'air (11) ont une forme de prisme tubulaire dont la section transversale a la forme d'un hexagone et dans lequel une pluralité d'unités de traitement de l'air sont assemblées en une structure en nid d'abeille, et dans lequel chaque lampe à excimère a la forme d'un tube allongé et est positionnée à égale distance des coins dudit hexagone, la distance (B) entre la surface de la lampe à excimère et la paroi intérieure de l'unité de traitement de l'air (4) étant inférieure ou égale à 2 cm, ladite distance étant mesurée dans la section transversale desdites unités de traitement de l'air, depuis la surface de la lampe jusqu'aux coins de l'hexagone.

2. Un système de traitement de l'air (1; 9; 10; 15; 16) selon la revendication 1, dans lequel les lampes à excimères (3) sont conçues pour émettre une longueur d'onde comprise entre 126 nm et 240 nm, de préférence environ 172 nm.

3. Un système de traitement de l'air (10) selon la revendication 1 ou 2, dans lequel la distance (B) entre la surface de la lampe à excimère et la paroi intérieure de l'unité de traitement de l'air (4) est de 1,5 cm ou de 1,0 cm, cette distance étant mesurée dans la section transversale desdites unités de traitement de l'air, depuis la surface de la lampe jusqu'aux coins de l'hexagone.

4. Un système de traitement de l'air (1; 9; 10; 15; 16) selon l'une quelconque des revendications précédentes, dans lequel les lampes à excimères (3) sont des tubes à excimère cylindriques allongés (5) ayant un axe longitudinal (X) disposé dans la direction du flux du boîtier de traitement de l'air (2) ou de l'unité de traitement de l'air (11).

5. Un système de traitement de l'air (15) selon l'une quelconque des revendications, dans lequel le système de traitement de l'air (15) comprend au moins un premier groupe de lampes à excimères (3a) agencées de manière à émettre une première longueur d'onde, et un second groupe de lampes à excimères (3b) agencées de manière à émettre une seconde longueur d'onde, et dans lequel la première et la seconde longueur d'onde sont différentes.

6. Un système de traitement de l'air (16) selon l'une quelconque des revendications précédentes, le système de traitement de l'air (16) comprenant au moins une première zone de traitement de l'air (17) comprenant des lampes à excimères (3a) agencées de manière à émettre une première longueur d'onde, et une seconde zone de traitement de l'air (18) comprenant des lampes à excimères (3b) agencées de manière à émettre une seconde longueur d'onde, et dans lequel la première et la seconde longueur d'onde sont différentes.

7. Un système de traitement de l'air (1; 9; 10; 15; 16) selon l'une des revendications 1 à 4, dans lequel toutes les lampes à excimères (3) du système de traitement de l'air (1; 9; 10; 15; 16) sont conçues pour émettre la même longueur d'onde.

8. Un système de traitement de l'air (1; 9; 10; 15; 16) selon l'une des revendications précédentes, dans lequel le système de traitement de l'air (1; 9; 10; 15; 16) est conçu pour faire fonctionner les lampes à excimères (3) avec une répétition d'impulsions.

9. Un système de traitement de l'air (1; 9; 10; 15; 16) selon la revendication 8, dans lequel les impulsions sont de courte durée, environ 100 ms ou moins, avec une fréquence porteuse comprise entre 10 Hz et 100 kHz, par exemple environ 20 kHz.

10. Un système de traitement de l'air (19) selon l'une quelconque des revendications précédentes, modifié en ce que le système de traitement de l'air comprend au moins un dispositif de filtrage (20), ledit système (19) étant agencé de manière à ce que la quasi-totalité de la surface du (des) dispositif(s) de filtrage faisant face aux lampes à excimères soit soumise aux photons émis par les lampes à excimères.

11. Un système de traitement de l'air (19) selon la revendication 10, dans lequel la distance directe (D) entre la surface des lampes à excimères (22) et la surface dudit au moins un dispositif de filtrage (23) faisant face aux lampes à excimères (3) est inférieure ou égale à 2 cm, par exemple 1,5 cm ou, de manière encore plus préférée, 1,0 cm.

12. Un procédé de traitement d'un flux d'air contaminé à l'aide du système de traitement de l'air (1; 9; 10; 15; 16) selon l'une des revendications 1 à 11.

13. Un procédé selon la revendication 12, dans lequel le flux d'air contaminé est exposé aux photons pendant une période d'au moins 1 ms.

14. Un procédé selon la revendication 12 ou 13, dans lequel le traitement est effectué à température ambiante, c'est-à-dire entre 10 et 50 °C environ, de préférence entre 20 et 25 °C environ.

15. Un système de chauffage et/ou de ventilation et/ou de climatisation comprenant le système de traitement de l'air (1; 9; 10; 15; 16) selon l'une des revendications 1 à 11.

16. Utilisation d'un système de traitement de l'air (1; 9; 10; 15; 16) selon l'une des revendications 1 - 11 pour traiter un flux d'air contaminé contenant des polluants sous forme de composés organiques, tels que les COV.
